# EUROPÄISCHE PATENTANMELDUNG

(11) **EP 0 652 204 A1**
(43) Veröffentlichungstag der Anmeldung: **10.05.1995**
(21) Anmeldenummer: 94116962.5
(22) Anmeldetag: 27.10.1994
(51) Int. Cl.: C07C 51/60

(54) **Verfahren zur Herstellung von Carbonsäurechloriden**

(30) Priorität: 05.11.1993 DE 4337785
(71) Anmelder: BASF Aktiengesellschaft, D-67063 Ludwigshafen (DE)
(72) Erfinder: Ettl, Roland, Dr., D-67454 Hassloch (DE); Reuther, Wolfgang, Dr., D-69118 Heidelberg (DE)

(57) **Zusammenfassung**

Herstellung von Carbonsäurechloriden der allgemeinen Formel I 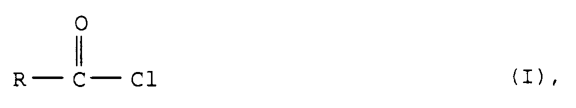
in der der Rest R für C₁-C₃₀-Alkyl, C₂-C₃₀-Alkenyl und C₂-C₃₀-Alkinyl steht, aus Carbonsäuren der allgemeinen Formel II 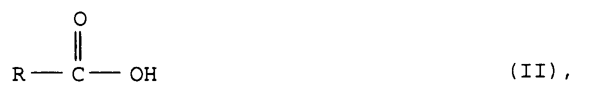
in der R die oben angegebene Bedeutung hat, und Phosgen COCl₂ (III) in Gegenwart eines Katalysator-Addukts aus Phosgen und einem N,N-disubstituiertem Formamid oder dessen Hydrochloriden der allgemeinen Formel IV 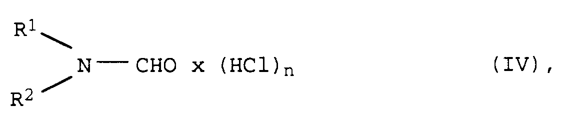
in der R¹ und R² unabhängig voneinander C₁-C₃-Alkyl oder gemeinsam eine C₄- oder C₅-Alkylenkette bedeuten, die gegebenenfalls durch ein Sauerstoffatom oder ein Stickstoffatom, das eine C₁-C₄-Alkylgruppe oder eine Formylgruppe trägt, unterbrochen sein kann, und n eine ganze Zahl von 0 bis 2 bedeutet, indem man
a) ein Formamid der Formel IV zu 0,1 bis 100 mol-% mit Phosgen belädt und anschließend
b) das so erhaltene Katalysator-Addukt mit einer Carbonsäure der Formel II umsetzt.

## Beschreibung

Die vorliegende Erfindung betrifft ein verbessertes Verfahren zur Herstellung von Carbonsäurechloriden der allgemeinen Formel I 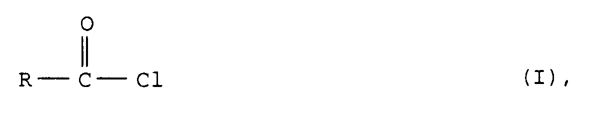
in der der Rest R für C₁-C₃₀-Alkyl, C₂-C₃₀-Alkenyl und C₂-C₃₀-Alkinyl steht, aus Carbonsäuren der allgemeinen Formel II 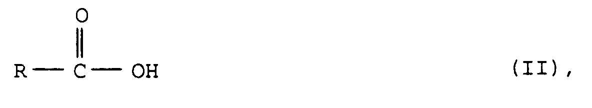
in der R die oben angegebene Bedeutung hat, und Phosgen COCl₂ (III) in Gegenwart eines Katalysator-Addukts aus Phosgen und einem N,N-disubstituiertem Formamid oder dessen Hydrochloriden der allgemeinen Formel IV 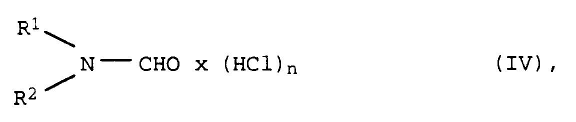
in der R¹ und R² unabhängig voneinander C₁-C₃-Alkyl oder gemeinsam eine C₄- oder C₅-Alkylenkette bedeuten, die gegebenenfalls durch ein Sauerstoffatom oder ein Stickstoffatom, das eine C₁-C₄-Alkylgruppe oder eine Formylgruppe trägt, unterbrochen sein kann, und n eine ganze Zahl von 0 bis 2 bedeutet.

Carbonsäurechloride sind gesuchte Zwischenprodukte für chemische Synthesen, insbesondere für die Verwendung in der Kosmetik- und Tensidchemie, sowie für Pharmazeutika und Pflanzenschutzmittel.

Die DE-A 40 12 781 beschreibt ein Verfahren zur Herstellung von Carbonsäurechloriden der Formel I aus Carbonsäuren der Formel II und Phosgen in Gegenwart von Katalysator-Addukten aus Phosgen und Formamiden der Formel IV. Nach der Lehre dieser Schrift werden die Carbonsäure und das Formamid gemeinsam vorgelegt und mit Phosgen versetzt, wobei die Reaktion zum Carbonsäurechlorid eintritt. Die Verfahrensprodukte fallen so in hoher Ausbeute an. Jedoch weist dieses Verfahren den Nachteil auf, daß die bei der Reaktion freigesetzten Gase Kohlendioxid CO₂ und Chlorwasserstoff gemeinsam freigesetzt werden, was einen Trennschritt für eine weitere Nutzung dieser Gase erforderlich macht. Weiterhin ist die Farbzahl ungesättigter Carbonsäurechloride nicht immer befriedigend, d.h. die Produkte sind durch unerwünschte Nebenprodukte teilweise stark gefärbt.

Es bestand die Aufgabe, ein Verfahren zur Herstellung von Carbonsäurechloriden der Formel I bereitzustellen, das die obengenannten Nachteile vermeidet.

Demgemäß wurde das oben definierte Verfahren gefunden, das dadurch gekennzeichnet ist, daß man
a) ein Formamid der Formel IV zu 0,1 - 100 mol-% mit Phosgen belädt und anschließend
b) das so erhaltene Katalysator-Addukt mit einer Carbonsäure der Formel II
umsetzt.

Ausgangsstoffe für das erfindungsgemäße Verfahren sind Carbonsäuren. Diese tragen geradkettige, verzweigte oder cyclische C₁-C₃₀-Alkylgruppen, z.B. Essigsäure, Propionsäure und 2-Ethyl-hexansäure. Bevorzugt sind aber geradkettige oder verzweigte ungesättigte Carbonsäuren, welche C₂-C₃₀-Alkenyl bzw. -Alkinylreste tragen. Besonders bevorzugt sind C₈-C₂₄-Alkenylfettsäuren wie Palmitoleinsäure, Ölsäure, Ricinensäure, Linolsäure, Linolensäure, Gadoleinsäure und Erucasäure.

Als Formamide der Formel IV kommen solche in Betracht, in denen die Reste R¹ und R² unabhängig voneinander C₁-C₃-Alkyl oder gemeinsam C₄- oder C₅-Alkenylkette bedeuten, die gegebenenfalls durch ein Sauerstoffatom oder ein Stickstoffatom, das eine C₁-C₃-Alkylgruppe oder eine Formylgruppe trägt, unterbrochen sein kann, und in denen n eine ganze Zahl von 0 bis 2 bedeutet. Bevorzugt sind N,N-Dialkylformamide wie N,N-Dimethylformamid, N,N-Methyl-ethylformamid und N,N-Diethylformamid. Wird der Katalysator mehrfach für die erfindungsgemäße Reaktion verwendet, erhöht sich erfahrungsgemäß der Anteil der Hydrochloride, d.h. n steht dann für 1 oder 2.

### Verfahrensschritt (a)

Ein Formamid der Formel IV wird erfindungsgemäß durch Einleiten von Phosgen zu 0,1 bis 100 mol-% beladen. In der Praxis haben sich besonders Beladungsgrade von 50 bis 95 mol-% bewährt, da diese einerseits durch eine befriedigend schnelle Umsetzung erhältlich sind und andererseits eine in der Regel ausreichend hohe Reaktionsgeschwindigkeit für die Reaktion zum Carbonsäurechlorid gewährleisten. Die Katalysatorbeladung wird im allgemeinen bei Temperaturen von 20 bis 70°C vorgenommen. Sie kann kontinuierlich durch die Messung der Dichte, Viskosität, Leitfähigkeit oder des Chloridgehaltes verfolgt werden.

### Verfahrensschritt (b)

Das gemäß Verfahrensschritt (a) erhaltene Katalysator-Addukt wird mit einer Carbonsäure der Formel II versetzt. Dabei kann die molare Menge an Carbonsäure der molaren Menge an Phosgen im Katalysator-Addukt entsprechen, sie kann aber auch bis zu 60 % geringer oder größer sein. Für den Erfolg des erfindungsgemäßen Verfahrens ist es unerheblich, ob die Carbonsäure zum Katalysatoraddukt oder das Katalysator-Addukt zur Carbonsäure dosiert wird. Aus praktischen Gründen ist jedoch die erste Ausführungsform bevorzugt.

Die Reaktionstemperatur beträgt im allgemeinen 10 bis 140°C, vorzugsweise 20 bis 100°C. Die Reaktion wird in der Regel bei Normaldruck durchgeführt, gegebenenfalls kann sie aber auch bei Drücken zwischen 0,01 bis 50 bar ausgeführt werden. Nach Beendigung der Zugabe der Carbonsäure kann das Reaktionsgemisch noch bis zu 2 h zur Vervollständigung der Umsetzung gerührt werden. Die Umsetzung kann in üblichen Reaktoren wie Rohrreaktoren, Schlangenreaktoren oder Rührkesseln diskontinuierlich oder kontinuierlich durchgeführt werden.

Die Aufarbeitung des Reaktionsproduktes erfolgt nach bekannten Methoden. So können kurzkettige Verfahrensprodukte extraktiv oder auch destillativ isoliert werden, wobei allerdings Temperaturen über 100°C vermieden werden sollten, da unter solchen Bedingungen der Katalysator zersetzt werden kann. In der Regel allerdings trennt sich das Verfahrensprodukt, insbesondere bei Carbonsäuren, deren Rest R mindestens 7 Kohlenstoffatome trägt, als leichtere Phase nach kurzer Zeit vom Katalysator ab und kann durch Phasentrennung isoliert werden, woran sich gegebenenfalls übliche Reinigungsverfahren wie Kristallisation oder Destillation anschließen können. Der Katalysator kann nach dem Abtrennen des Verfahrensproduktes beliebig oft für weitere Umsetzungen wieder eingesetzt werden.

Das erfindungsgemäße Verfahren erlaubt die einfache Herstellung und Isolierung von hochreinen, phosgenfreien Carbonsäurechloriden. Dabei fallen im Verfahrensschritt (a) CO₂, in Verfahrensschritt (b) Chlorwasserstoff an, die für andere Umsetzungen genutzt werden können. Weiterhin weisen erfindungsgemäß hergestellte ungesättigte Carbonsäurechloride eine niedrige Iodzahl auf, d.h. sie sind nur minimal gefärbt.

### Beispiele

Die Bestimmung der Iodfarbzahl (JFZ) erfolgte nach DIN 55 945.

### Verfahrensschritt (a)

1.1 Beladung des Katalysators
   In einem Rohrreaktor wurden 4 mol (550 g) Diethylformamid-Hydrochlorid vorgelegt. Bei 60°C wurde solange Phosgen zugeleitet, bis die Leitfähigkeit einen Wert von 14 mS/cm erreichte, was einem Beladungsgrad von 95 mol-% entspricht.

### Verfahrensschritt (b)

1.2 Herstellung von Ölsäurechlorid
   4 mol (1.088 g) Ölsäure wurden innerhalb von 45 min bei 40°C zu dem nach Beispiel 1.1 hergestellten Katalysator dosiert. Nach Ende der Zugabe wurde 0,5 h nachgerührt und dann 0,5 h zur Phasentrennung stehengelassen. Das Ölsäurechlorid konnte als Oberphase abgehoben werden.
   Man erhielt in einer Ausbeute von 97 % Ölsäurechlorid mit einer JFZ von 10.
1.3 Herstellung von Myristoleinsäurechlorid
   In Analogie zu Beispiel 1.2 wurden 2 mol Myristoleinsäure über 60 min zum Katalysator nach Beispiel 1.1 gegeben und aufgearbeitet.
   Die Ausbeute betrug 95 % mit einer JFZ von 15.
1.4 Herstellung von Erucasäurechlorid
   2 mol Erucasäure wurden in Analogie zu Beispiel 1.3 umgesetzt und aufgearbeitet.
   Die Ausbeute betrug 98 % mit einer JFZ von 10.
1.5 Herstellung von Linolsäurechlorid
   2 mol Linolsäure wurden in Analogie zu Beispiel 1.3 über 30 min zum Katalysator gegeben und aufgearbeitet.
   Die Ausbeute betrug 95 % mit einer JFZ von 36.

## Patentansprüche

1. Verfahren zur Herstellung von Carbonsäurechloriden der allgemeinen Formel I 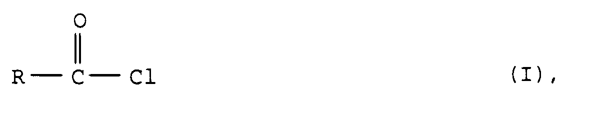 in der der Rest R für C₁-C₃₀-Alkyl, C₂-C₃₀-Alkenyl und C₂-C₃₀-Alkinyl steht, aus Carbonsäuren der allgemeinen Formel II 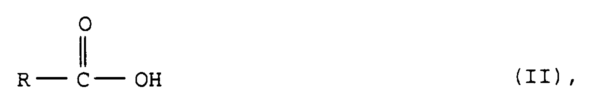 in der R die oben angegebene Bedeutung hat, und Phosgen COCl₂ (III) in Gegenwart eines Katalysator-Addukts aus Phosgen und einem N,N-disübstituiertem Formamid oder dessen Hydrochloriden der allgemeinen Formel IV 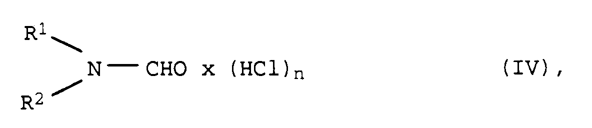 in der R¹ und R² unabhängig voneinander C₁-C₃-Alkyl oder gemeinsam eine C₄- oder C₅-Alkylenkette bedeuten, die gegebenenfalls durch ein Sauerstoffatom oder ein Stickstoffatom, das eine C₁-C₄-Alkylgruppe oder eine Formylgruppe trägt, unterbrochen sein kann, und n eine ganze Zahl von 0 bis 2 bedeutet, dadurch gekennzeichnet, daß man
a) ein Formamid der Formel IV zu 0,1 bis 100 mol-% mit Phosgen belädt und anschließend
b) das so erhaltene Katalysator-Addukt mit einer Carbonsäure der Formel II umsetzt.

2. Verfahren nach Anspruch 1, dadurch gekennzeichnet, daß man als Formamid der Formel IV N,N-Dimethylformamid, N,N-Methylethylformamid oder N,N-Diethylformamid verwendet.

3. Verfahren nach Anspruch 1 oder 2, dadurch gekennzeichnet, daß man als Carbonsäuren der Formel II C₈-C₂₄-Alkenylfettsäuren verwendet.

4. Verfahren nach den Ansprüchen 1 bis 3, dadurch gekennzeichnet, daß man das Carbonsäurechlorid I durch Phasentrennung aus dem Reaktionsgemisch isoliert.
